Europäisches Patentamt

European Patent Office

Office européen des brevets

(11) Veröffentlichungsnummer: **0 1 1 4 330**
**B1**

(12) **EUROPÄISCHE PATENTSCHRIFT**

(45) Veröffentlichungstag der Patentschrift:
02.10.85

(21) Anmeldenummer: 83112746.9

(22) Anmeldetag: 17.12.83

(51) Int. Cl.⁴: **C 07 C 51/58, C 07 C 53/40,**
**C 07 C 53/42, C 07 C 57/72**

(54) Verfahren zur Herstellung von Carbonsäurechloriden und -bromiden.

(30) Priorität: 29.12.82 DE 3248468

(43) Veröffentlichungstag der Anmeldung:
01.08.84 Patentblatt 84/31

(45) Bekanntmachung des Hinweises auf die Patenterteilung:
02.10.85 Patentblatt 85/40

(84) Benannte Vertragsstaaten:
AT BE CH DE FR GB IT LI NL

(56) Entgegenhaltungen:
DE - A - 2 648 134

(73) Patentinhaber: HOECHST AKTIENGESELLSCHAFT,
Postfach 80 03 20, D-6230 Frankfurt am Main 80 (DE)

(72) Erfinder: Erpenbach, Heinz, Dr., Oberbuschweg 22,
D-5000 Köln 50 (DE)
Erfinder: Gehrmann, Klaus, Dr.,
Geschwister-Scholl-Strasse 32, D-5042 Erftstadt (DE)
Erfinder: Lork, Winfried,
Siegfried-von-Westerburg-Strasse 14, D-5042 Erftstadt
(DE)
Erfinder: Prinz, Peter, Von-Geyr-Ring 104, D-5030 Hürth
(DE)

## Beschreibung

Die Erfindung betrifft ein Verfahren zur Herstellung von Carbonsäurechloriden oder -bromiden durch Umsetzen von Alkylchloriden oder -bromiden mit 1–6 C-Atomen oder Arylchloriden oder -bromiden mit Kohlenmonoxid, gegebenenfalls in Mischung mit bis zu 30 Vol% Wasserstoff, unter praktisch wasserfreien Bedingungen bei Temperaturen von 350 bis 575 K und Drücken von 1 bis 300 bar in Gegenwart eines Katalysatorsystems, welches mindestens ein Edelmetall der Gruppe Rh, Ir oder Pd oder dessen Verbindung, Jod und/oder dessen Verbindungen und gegebenenfalls ein inertes organisches Lösungsmittel enthält, welches dadurch gekennzeichnet ist, daß das Katalysatorsystem zusätzlich eine Verbindung der Elemente der Gruppen I–III des Periodensystems sowie Essigsäure enthält. Bevorzugt arbeitet man in Gegenwart eines Katalysatorsystems, das Verbindungen des Lithiums und/oder Kaliums und/oder Magnesiums und/oder Calciums und/oder Aluminiums enthält. Weiterhin erweist sich der Einsatz eines Katalysatorsystems als vorteilhaft, das über die genannten Verbindungen hinaus eine oder mehrere Verbindungen der unedlen Metalle Ce, Ti, Zr, Hf, Ge, Sn, Pb, V, Nb, Ta, As, Sb, Bi, Cr, Mo, W, Mn, Re, Fe, Co, Ni enthält.

Die DE-OS 3 016 900 beschreibt ein Verfahren zur Herstellung von Acetylchlorid durch Umsetzung von Methylchlorid mit Kohlenmonoxid unter praktisch wasserfreien Bedingungen bei Temperaturen von 350 bis 575 K und Drücken von 1 bis 300 bar in Gegenwart eines Katalysatorsystems, welches mindestens eines der Edelmetalle Rhodium, Palladium, Iridium oder deren Verbindungen, Jod und/oder dessen Verbindungen, ggf. Trialkylphosphinoxid oder Triarylphosphinoxid, sowie ein inertes organisches Lösungsmittel enthält, welches dadurch gekennzeichnet ist, daß man in Gegenwart eines Katalysatorsystems arbeitet, welches n-Heptan als inertes Lösungsmittel und zusätzlich Methyltrialkylphosphoniumjodid und/oder Methyltriarylphosphoniumjodid und mindestens eine Verbindung des Chroms, Molybdäns oder Wolframs enthält.

Die DE-OS 3 035 201 wiederum beansprucht ein Verfahren zur Herstellung von Carbonsäurehalogeniden durch Umsetzung von Alkylhalogeniden mit 1–6 C-Atomen oder Arylhalogeniden und Kohlenmonoxid in Gegenwart eines Katalysatorsystems, welches mindestens eines der Edelmetalle Rhodium, Palladium, Iridium oder deren Verbindungen, Jod und/oder dessen Verbindungen, Methyltrialkyl- und/oder Methyltriarylphosphoniumjodid, ggf. Trialkylphosphinoxid oder Triarylphosphinoxid und ggf. ein inertes organisches Lösungsmittel enthält, welches dadurch gekennzeichnet ist, daß man dem Reaktionsgemisch 0,02 bis 0,75 Mol Wasserstoff je Mol Kohlenmonoxid zufügt. Als Copromotoren können dem Katalysatorsystem zusätzlich eine oder mehrere Verbindungen der unedlen Metalle Ce, Ti, Zr, Hf, Ge, Sn, Pb, V, Nb, Ta, As, Sb, Bi, Cr, Mo, W, Mn, Re, Fe, Co, Ni zugesetzt werden.

Durch den Zusatz von Wasserstoff wird vor allem eine gleichbleibende Aktivität des Carbonylierungskatalysators aufrechterhalten.

Die vorliegende Erfindung ermöglicht es, die Carbonylierung eines Alkyl- oder Arylchlorids oder -bromids in einem Katalysatorsystem durchzuführen, bei dem im Gegensatz zum Stand der Technik auf den Zusatz organischer Phosphorverbindungen gänzlich verzichtet werden kann. Statt dessen werden als Promotoren Verbindungen der Elemente der Gruppen I–III des Periodensystems verwendet. Dabei ist der Zusatz von Essigsäure zum Katalysatorgemisch unerläßlich. So gelingt es, ein Reaktionssystem gleichbleibender Aktivität zu erhalten, das die Gewinnung von Carbonsäurechloriden oder -bromiden in guten Raum-Zeit-Ausbeuten zuläßt.

Die Edelmetalle sowie die genannten unedlen Metalle werden bevorzugt in Form ihrer Chloride (z. B. $RhCl_3 \cdot 3 H_2O$, $CrCl_3 \cdot 6 H_2O$), Acetate, Carbonate, Carbonyle (z. B. $[Re(CO)_5]_2$, $W(CO)_6$) oder als Komplexverbindungen (z. B. $[Rh(CO)_2Cl]_2$, $[Pd(CO)_2J]_2$) eingesetzt.

Die Metallverbindungen der Gruppen I–III des Periodensystems der Elemente werden bevorzugt in Form ihrer Acetate, Carbonate oder Jodide verwendet. Als Jodverbindung wird bevorzugt Methyljodid, aber auch Ethyljodid oder Jodwasserstoff eingesetzt.

Die Essigsäure wird vorzugsweise in reiner Form oder auch in Form eines Gemisches mit Essigsäureanhydrid zugesetzt.

Das Katalysatorsystem Edelmetall(-verbindung)/Unedelmetall(-verbindung)/Jod(-verbindung)/Metallverbindung der Gruppen I–III des Periodensystems/Essigsäure/organisches Lösungsmittel wird bevorzugt im Molverhältnis

$$1 : (0-8) : (1-100) : (1-100) : (50-500) : (0-500)$$

eingesetzt.

Je Mol des Ausgangsstoffes Alkyl- bzw. Arylchlorid oder -bromid wird vorzugsweise 0,0001–0,01 Mol Edelmetall(-verbindung) und 0,05–2 Mol Essigsäure eingesetzt.

Das eingesetzte Kohlenmonoxid kann neben Wasserstoff auch unter den Reaktionsbedingungen inerte Gase wie Stickstoff, Methan, Ethan, Propan enthalten. Als inertes Lösungsmittel kann ggf. Hexan, Heptan, Octan, Toluol, Xylol oder Essigsäureanhydrid eingesetzt werden.

Die Umsetzung gemäß der Erfindung erfolgt bevorzugt bei 20–180 bar und 150–250°C (423–523 K).

Das Verfahren der Erfindung kann sowohl in diskontinuierlicher als auch in kontinuierlicher Betriebsweise durchgeführt werden.

2

## Beispiel 1

a) 1,38 g RhCl₃ · 3 H₂O, 5,55 g CrCl₃ · 6 H₂O, 6,93 g Li(CH₃COO), 26,1 g Methyljodid, 102,9 Essigsäure und 105,9 g Methylchlorid werden in einen Autoklaven aus korrisionsbeständigem Edelstahl eingefüllt, zunächst mit einem Wasserstoff-Partialdruck von 7,5 bar und dann mit einem CO-Partialdruck von 75 bar versehen (Gesamtdruck 82,5 bar). Nach einer Reaktionszeit von 1 Stunde bei 453 K wird das Reaktionsprodukt dem Autoklaven entnommen und analysiert. Es werden 84 g Acetylchlorid (51,0% d. Th.) ermittelt, woraus sich eine Raum-Zeit-Ausbeute von 338 g CH₃COOI/l Reaktionsvolumen (Rv) · h bzw. eine Katalysatorleistung von 156 g CH₃COOI/g Rh · h errechnet.

b) (Vergleichsversuch)
1,38 g RhCl₃ · 3 H₂O, 5,55 g CrCl₃ · 6 H₂O, 26,1 g Methyljodid, 102,9 g Essigsäure und 105,9 g Methylchlorid werden in einen Autoklaven aus korrosionsbeständigem Edelstahl eingehüllt, zunächst mit einem Wasserstoff-Partialdruck von 7,5 bar und dann mit einem CO-Partialdruck von 75 bar versehen (Gesamtdruck 82,5 bar). Nach einer Reaktionszeit von 1 Stunde bei 453 K zeigt ein unveränderter Druck im Autoklaven, daß bisher kein CO-Umsatz stattgefunden hat. Erst nach Steigerung der Temperatur auf 463 K ist ein geringer Druckabfall im Autoklaven zu beobachten. Nach einer Gesamtreaktionszeit von 4,7 Stunden wird das Reaktionsprodukt dem Autoklaven entnommen und analysiert. Es werden 23,5 g Acetylchlorid (14,3% d. Th.) gefunden, woraus sich eine Raum-Zeit-Ausbeute von 21 g CH₃COCl/l Rv · h bzw. eine Katalysatorleistung von 9,3 g CH₃COCl/g Rh · h ergibt.

## Beispiel 2

1,38 g RhCl₃ · 3 H₂O, 6,93 g Li(CH₃COO), 26,1 g Methyljodid 102,9 g Essigsäure und 105,9 g Methylchlorid werden in einen Autoklaven aus korrosionsbeständigem Edelstahl eingefüllt, unter einen Wasserstoffdruck von 7,5 bar und dann unter einen CO-Druck von 75 bar (Gesamtdruck 82,5 bar) gesetzt. Nach einer Reaktionszeit von 1 Stunde bei 453 K wird ein Reaktionsprodukt mit 80,5 g Acetylchlorid (48,9% d. Th.) erhalten, woraus sich eine Raum-Zeit-Ausbeute von 331 g CH₃COCl/l Rv · h bzw. eine Katalysatorleistung von 149 g CH₃COCl/g Rh · h ergibt.

## Beispiel 3

1,38 g RhCl₃ · 3 H₂O, 10,31 g K(CH₃COO), 26,1 g Methyljodid 102,9 g Essigsäure und 105,9 g Methylchlorid werden in einen Autoklaven aus korrosionsbeständigem Edelstahl eingefüllt, zunächst mit einem Wasserstoff-Partialdruck von 7,5 bar und dann mit einem CO-Partialdruck von 75 bar versehen. Nach einer Reaktionszeit von 1 Stunde bei 453 K werden im Reaktionsprodukt 40 g Acetylchlorid (24,3% d. Th.) gefunden. Es errechnet sich daraus eine Raum-Zeit-Ausbeute von 162 g CH₃COCl/l Rv · h bzw. eine Katalysatorleistung von 74 g CH₃COCl/g Rh · h.

## Beispiel 4

1,38 g RhCl₃ · 3 H₂O, 10,40 g Mg(CH₃COO)₂ · 4 H₂O, 26,1 g Methyljodid, 102,9 g Essigsäure und 105,9 g Methylchlorid werden in einen Autoklaven aus korrosionsbeständigem Edelstahl gegeben und unter einen Wasserstoffdruck von 7,5 bar und einen CO-Druck von 75 bar (Gesamtdruck 82,5 bar) gesetzt. Nach einer Reaktionszeit von 1 Stunde bei 453 K werden im Reaktionsprodukt 63 g Acetylchlorid (38,3% d. Th.) bestimmt. Daraus ergibt sich eine Raum-Zeit-Ausbeute von 225 g CH₃COCl/l Rv · h bzw. eine Katalysatorleistung von 117 g CH₃COCl/g Rh · h.

## Beispiel 5

1,38 g RhCl₃ · 3 H₂O, 20,39 g Ca(CH₃COO)₂ · 2 H₂O, 26,1 g Methyljodid, 102,9 g Essigsäure und 105,9 g Methylchlorid werden in einen Autoklaven aus korrosionsbeständigem Edelstahl eingefüllt und danach unter einen Wasserstoffdruck von 7,5 bar und einen CO-Druck von 75 bar (Gesamtdruck 82,5 bar) gesetzt. Nach einer Reaktionszeit von 0,5 Stunden bei 453 K werden 61 g Acetylchlorid (37,1% d. Th.) gefunden. Es ergibt sich somit eine Raum-Zeit-Ausbeute von 475 g CH₃COCl/l Rv · h bzw. eine Katalysatorleistung von 226 g CH₃COCl/g Rh · h.

## Beispiel 6

1,38 g $RhCl_3 \cdot 3 H_2O$, 4,69 g $ZrO(CH_3COO)_2$, 17,0 g $Al(OH) (CH_3COO)_2$, 26,1 g Methyljodid, 102,9 g Essigsäure und 105,9 g Methylchlorid werden in einen Autoklaven aus korrosionsbeständigem Edelstahl eingefüllt und danach einem Wasserstoffdruck von 7,5 bar und einem CO-Druck von 75 bar (Gesamtdruck 82,5 bar) ausgesetzt. Nach einer Reaktionszeit von 0,5 Stunden bei 453 K werden 54 g Acetylchlorid (32,8% d. Th.) analysiert. Es ergibt sich somit eine Raum-Zeit-Ausbeute von 419 g $CH_3COCl/l$ Rv · h bzw. eine Katalysatorleistung von 200 g $CH_3COCl/g$ Rh · h.

## Beispiel 7

1,38 g $RhCl_3 \cdot 3 H_2O$, 6,24 g $[Re(CO)_5]_2$, 6,93 g $Li(CH_3COO)$, 26,1 g Methyljodid, 102,9 g Essigsäure und 199,1 g Methylbromid werden in einen Autoklaven aus korrosionsbeständigem Edelstahl eingefüllt und danach ein Wasserstoffdruck von 7,5 bar und ein CO-Druck von 75 bar (Gesamtdruck 82,5 bar) aufgegeben. Nach einer Reaktionszeit von 0,75 Stunden bein 453 K werden 87 g Acetylbromid (33,7% d. Th.) ermittelt. Es ergibt sich somit eine Raum-Zeit-Ausbeute von 339 g $CH_3COBr/l$ Rv · h bzw. eine Katalysatorleistung von 215 g $CH_3COBr/g$ Rh · h.

## Beispiel 8

1,38 g $RhCl_3 \cdot 3 H_2O$, 5,56 g Vanadylacetylacetonat $[VO(C_5H_7O_2)_2]$, 17,0 g $Al(OH)(CH_3COO)_2$, 26,1 g Methyljodid, 102,9 g Essigsäure und 129 g Ethylchlorid werden in einen Autoklaven aus korrosionsbeständigem Edelstahl eingefüllt und danach mit einem Wasserdruck von 7,5 bar und einem CO-Druck von 75 bar (Gesamtdruck 82,5 bar) versehen. Nach einer Reaktionszeit von 3 Stunden bei 458 K werden 15 g Propionylchlorid (8,1% d. Th.) gefunden. Es ergibt sich somit eine Raum-Zeit-Ausbeute von 18 g $CH_3CH_2COCl/l$ Rv · h bzw. eine Katalysatorleistung von 9,3 g $CH_3CH_2COCl/g$ Rh · h.

## Beispiel 9

1,38 g $RhCl_3 \cdot 3 H_2O$, 3,55 g $Ni(CO)_4$, 10,20 g $Ca(CH_3COO)_2 \cdot 2 H_2O$, 26,1 g Methyljodid, 102,9 g Essigsäure und 113,9 g Benzylchlorid werden in einen Autoklaven aus korrosionsbeständigem Edelstahl eingefüllt und unter einen Wasserstoffdruck von 7,5 bar und einen CO-Druck von 75 bar (Gesamtdruck 82,5 bar) gesetzt. Nach einer Reaktionszeit von 1 Stunde bei 453 K werden 44,5 g Phenyl-essigsäurechlorid (32,0% d. Th.) analysiert. Es ergibt sich eine Raum-Zeit-Ausbeute von 172 g $C_6H_5CH_2COCl/l$ Rv · h bzw. eine Katalysatorleistung von 83 g $C_6H_5CH_2COCl/g$ Rh · h.

## Beispiel 10

1,11 g $PdCl_2 \cdot 2 H_2O$, 4,69 g $ZrO(CH_3COO)_2$, 20,39 g $Ca(CH_3COO)_2 \cdot 2 H_2O$, 26,1 g Methyljodid, 102,9 g Essigsäure und 105,9 g Methylchlorid werden in einen Autoklaven aus korrosionsbeständigem Edelstahl eingesetzt und einem Wasserstoffdruck von 7,5 bar sowie einem CO-Druck von 75 bar (Gesamtdruck 82,5 bar) ausgesetzt. Nach einer Reaktionszeit von 1 Stunde bei 453 K werden 47 g Acetylchlorid (28,6% d. Th.) ermittelt. Es ergibt sich somit eine Raum-Zeit-Ausbeute von 180 g $CH_3COCl/l$ Rv · h bzw. eine Katalysatorleistung von 85 g $CH_3COCl/g$ Pd · h.

## Beispiel 11

1,56 g $IrCl_3$, 6,93 g $Li(CH_3COO)$, 26,1 g Methyljodid, 102,9 g Essigsäure und 105,9 g Methylchlorid werden in einen Autoklaven aus korrosionsbeständigem Edelstahl eingefüllt und danach ein Wasserstoffdruck von 7,5 bar sowie ein CO-Druck von 75 bar (Gesamtdruck 82,5 bar) aufgegeben. Nach einer Reaktionszeit von 3 Stunden bei 463 K werden 31 g Acetylchlorid (18,8% d. Th.) gefunden. Es ergibt sich somit eine Raum-Zeit-Ausbeute von 42,5 g $CH_3COCl/l$ Rv · h bzw. eine Katalysatorleistung von 10,3 g $CH_3COCl/g$ Ir · h.

## Beispiel 12

1,38 g $RhCl_3 \cdot 3 H_2O$, 6,93 g $Li(CH_3COO)$, 26,1 g Methyljodid, 52 g Essigsäure, 52 g Essigsäureanhydrid und 105,9 g Methylchlorid werden in einen Autoklaven aus korrosionsbeständigem Edelstahl eingefüllt und danach 7,5 bar Wasserstoff sowie 75 bar CO (Gesamtdruck 82,5 bar) aufgedrückt. Nach

4

einer Reaktionszeit von 1 Stunde bei 453 K werden 85 g Acetylchlorid (51,6% d. Th.) analysiert. Es ergibt sich somit eine Raum-Zeit-Ausbeute von 348 g $CH_3COCl/l$ Rv · h bzw. eine Katalysatorleistung von 158 g $CH_3COCl/g$ Rh · h.

## Patentansprüche

1. Verfahren zur Herstellung von Carbosäurechloriden oder -bromiden durch Umsetzung von Alkylchloriden oder -bromiden mit 1—6 C-Atomen oder Arylchloriden oder -bromiden mit Kohlenmonoxid, gegebenenfalls in Mischungen mit bis zu 30 Vol% Wasserstoff, unter praktisch wasserfreien Bedingungen bei Temperaturen von 350 bis 575 K und Drücken von 1 bis 300 bar in Gegenwart eines Katalysatorsystems, welches mindestens ein Edelmetall der Gruppe Rh, Ir oder Pd oder dessen Verbindung, Jod und/oder dessen Verbindungen und gegebenenfalls ein inertes organisches Lösemittel enthält, dadurch gekennzeichnet, daß das Katalysatorsystem zusätzlich eine Verbindung der Elemente der Gruppen I—III des Periodensystems sowie Essigsäure enthält.

2. Verfahren nach Anspruch 1, dadurch gekennzeichnet, daß das Katalysatorsystem mindestens eine Verbindung der Metalle Lithium, Kalium, Magnesium, Calcium oder Aluminium enthält.

3. Verfahren nach Anspruch 1 oder 2, dadurch gekennzeichnet, daß das Katalysatorsystem zusätzlich eine oder mehrere Verbindungen der unedlen Metalle Ce, Ti, Zr, Hf, Ge, Sn, Pb, V, Nb, Ta, As, Sb, Bi, Cr, Mo, W, Mn, Re, Fe, Co, Ni enthält.

4. Verfahren nach einem der Ansprüche 1—3, dadurch gekennzeichnet, daß man das Katalysatorsystem Edelmetall(-Verbindung)/Unedelmetallverbindung/Jod(-verbindung)/Metallverbindung der Gruppen I—III /Essigsäure/organ. Lösemittel im Molverhältnis

$$1 : (0-8) : (1-100) : (1-100) : (50-500) : (0-500)$$

einsetzt.

## Claims

1. Process for making carboxylic acid chlorides or bromides by reacting an alkyl chloride or bromide having 1 to 6 carbon atoms or aryl chloride or bromide with carbon monoxide, optionally in admixture with up to 30 volume% hydrogen, under practcally anhydrous conditions at temperatures to 350 to 575 K pressures of 1 to 300 bars in the presence of a catalyst system containing at least one noble metal belonging to the group of Rh, Ir and Pd or its compounds, iodine and/or its compounds, and optionally an inert organic solvent, characterized in that the catalyst system additionally contains a compound of the elements belonging to groups I to III of the Periodic System, and acetic acid.

2. Process as claimed in claim 1, wherein the catalyst system contains at least one compound of the metals lithium, potassium, magnesium, calcium or aluminium.

3. Process as claimed in claim 1 or 2, wherein the catalyst system additionally contains one or more compounds of the non-noble metals Ce, Ti, Zr, Hf, Ge, Sn, Pb, V, Nb, Ta, As, Sb, Bi, Cr, Mo, W, Mn, Re, Fe, Co, Ni.

4. Process as claimed in any of claims 1—3, wherein a catalyst system containing the noble metal (-compound)/non-noble compound/iodine(-compound)/metal compound of groups I to III/acetic acid/organic solvent in a molar ratio of

$$1 : (0-8) : (1-100) : (1-100) : (50-500) : (0-500)$$

is used.

## Revendications

1. Procédé de préparation de chlorures ou bromures d'acides carboxyliques par réaction de chlorures ou bromures d'alkyles en $C_1—C_6$ ou de chlorures ou bromures d'aryles avec le monoxyde de carbone, éventuellement en association avec jusqu'à 30% en volume d'hydrogène, dans des conditions pratiquement anhydres à des températures de 350—575 K et sous des pressions de 1—300 bars en présence d'un système catalytique contenant au moins un métal noble du groupe Rh, Ir et Pd ou son composé, de l'iode et/ou ses composés et, éventuellement, un solvant organique inerte, caractérisé en ce que le système catalytique contient comme composant supplémentaire un composé des éléments appartenant aux groupes I—III de la Classification Périodique ainsi que l'acide acétique.

2. Procédé selon la revendication 1, caractérisé en ce que le système catalytique contient au moins un composé des métaux lithium, potassium, magnésium, calcium ou aluminium.

3. Procédé selon la revendication 1 ou 2, caractérisé en ce que le système catalytique contient en

**0 114 330**

outre un ou plusieurs composés des métaux non nobles Ce, Ti, Zr, Hf, Ge, Sn, Pb, V, Nb, Ta, As, Sb, Bi, Cr, Mo, W, Mn, Re, Fe, Co, Ni.

4. Procédé selon l'une des revendications 1 à 3, caractérisé en ce que l'on utilise le système catalytique (composé de) métal noble/composé de métal non noble/(composé d') iode/composé de métal des groupes I—III/acide acétique/solvant organique dans les proportions molaires

1 : (0—8) : (1—100) : (1—100) : (50—500) : (0—500).